# EUROPEAN PATENT SPECIFICATION

(11) **EP 0 866 685 B1**
(45) Date of publication and mention of the grant of the patent: **16.04.2003**
(21) Application number: 96936894.3
(22) Date of filing: 22.10.1996
(51) Int. Cl.: A61K 7/32, A61K 7/34, A61K 7/36, A61K 7/38, A61K 9/10, A61K 31/20, A61K 31/74

(54) **LIQUID DEODORANT COMPOSITIONS**
FLÜSSIGE DEODORANTIEN
COMPOSITIONS DEODORANTES LIQUIDES

(30) Priority: 25.11.1995 GB 9524158
(43) Date of publication of application: 30.09.1998
(73) Proprietor: THE PROCTER & GAMBLE COMPANY, Cincinnati, Ohio 45202 (US)
(72) Inventor: MEADOWS, Grant, Robert, Egham, Surrey TW 20 9NG (GB); MEYER, Gerard, Bernard, Walton-on-Thames, Surrey KT1 25AZ (GB)
(74) Representative: Wilding, Richard Alan
(86) International application number: US9617002
(87) International publication number: WO97019674

(56) References cited:
- EP-A- 0 674 899
- US-A- 4 659 564
- US-A- 5 409 694

## Description

The present invention relates to liquid deodorant compositions comprising the deodorant active zinc phenolsulfonate. These compositions are particularly suitable for use as aerosols.

### BACKGROUND OF THE INVENTION

Deodorant liquid compositions have become part of many people's personal care and grooming regimen. These compositions may be delivered to the body via a variety of devices such as aerosol sprays, pump sprays, and liquid applicators such as roll-on devices.

The formulation of deodorant products is a delicate balancing of perfume, deodorant active, cosmetic factors and skin irritation factors. The resulting product is often a consumer acceptable product but not an outstanding product in all areas.

Historically, perfumes were used to mask body odours. Today, deodorant compositions not only comprise perfumes, but, also, antimicrobials, herein referred to as deodorant actives. The deodorant actives enhance the ability of the perfumes to mask malodors by destroying the microbes which abide on the skin. The microbes attack sweat gland secretions thus causing the formation of malodorous fatty acids. It is through the combined efforts of the perfume and actives that deodorants are effective.

Aerosol deodorants are typically about 20% to 40% propellant, 50-70% volatile monohydric alcohol such as ethanol, and up to about 10% propylene glycol, a deodorant active and fragrance. Typically, only about 20% to 60% of the sprayable contents actually reach the skin since the liquefied hydrocarbon propellant vaporises as it is sprayed.

Perceived cosmetics can be important to consumers in a liquid deodorant composition, as can odor stability. Aerosol deodorants have gained wide consumer acceptance due to their excellent cosmetic characteristics.

In addition to the consumer desirable attributes of odor masking and cosmetic characteristics, liquid deodorant products which are mild and non-irritating in character and have a dry, non-sticky or non-greasy skin feel are desired.

Stick deodorant compositions containing high levels of dipropylene glycol are known to deliver good non-sticky, non-greasy skin feel attributes. Use of high levels of dipropylene glycol in liquid deodorant compositions, such as aerosols, is not common. It is thought that incorporation of diproylene glycol at high levels could produce poor skin feel attributes such as greasy, wet skin feel, particularly when sprayed.

US-A-5,409,694 discloses liquid deodorant compositions comprising polyhydric alcohols, monohydric alcohols, a deodorant active and silicone emollients.

Applicant has now found that it is possible to formulate mild liquid deodorant compositions comprising high levels of dipropylene glycol with C₁-C₄ alcohol in particular levels and ratios, in combination with zinc phenol sulphonate and silicone emollients which deliver excellent efficacy and non-greasy, non-tacky skin feel attributes and demonstrate good stability characteristics.

### SUMMARY OF THE INVENTION

The liquid deodorant compositions according to the present invention comprise:
a) from 10% to 40% by weight, of a polyhydric alcohol selected from propylene glycol, dipropylene glycol, polypropylene glycols having three or more propylene monomer units, ethylene glycol, diethylene glycol, hexylene glycol, butylene glycol, and mixtures thereof;
b) from 10% to 70% by weight, of C₁-C₄ monohydric alcohol;
c) from 0.1% to 4% by weight, of zinc phenol sulphonate; and
d) from 0.1% to 10% by weight, of at least one silicone containing emollient;

These deodorant compositions are particularly useful for delivery by aerosol devices. However, other liquid applicators may be used.

### DETAILED DESCRIPTION OF THE INVENTION

The components utilised in the present invention are described in detail below.

All percentages and ratios herein are by weight unless otherwise indicated.
Compositions of the present invention are preferably single phase solutions which remain stable over a typical shelf life of said compositions.

The compositions of the present invention include, as an essential component, at least one polyhydric alcohol. Polyhydric alcohols are valuable in the compositions according to the present invention for enhanced deodorancy, fragrance fixing and emolliency attributes.

Polyhydric alcohols suitable for use herein include propylene glycol, dipropylene glycol, polypropylene glycols having three or more propylene units, ethylene glycol, diethylene glycol, hexylene glycol, butylene glycol and mixtures thereof. Preferred for use herein is dipropylene glycol.

Polyhydric alcohol is present in the compositions according to the present invention at a level of from 10% to 40%, preferably from 10% to 30%, more preferably from 15% to 25% by weight.

Applicant has found that at levels lower than 5 % and greater than 40% by weight the compositions can either have poor fragrance longevity or undesirable wet skin feel attributes.

Monohydric alcohol is a further essential component of the compositions according to the present invention. The C₁-C₄ monohydric alcohols are used in the liquid deodorant compositions of the present invention as a liquid solvent vehicle. Applicant has also found that C₁-C₄ monohydric alcohol is particularly valuable in the compositions according to the present invention for the delivery of desirable non-sticky, non-tacky, non-greasy skin feel attributes when combined with polyhydric alcohol in a weight ratio of from about 1:4 to about 4:1, preferably from about 1:3 to about 3:1 more preferably from about 1:3 : 1:1.

C₁-C₄ monohydric alcohols are used in the liquid deodorant compositions of the present invention at levels of from 15% to 70%, preferably from 20% to 60%, more preferably 25% to 50% and especially from 30% to 45% by weight.

Examples of suitable C₁-C₄ monohydric alcohols include methanol, ethanol, isopropanol and mixtures thereof. The preferred C₁-C₄ monohydric alcohol for use in the present compositions is ethanol. A suitable commercially available ethanol is Denatured Ethanol B-100 available from B.P. Chemicals Limited.

A further essential component of the compositions according to the present inventions is deodorant active. The particular deodorant active of the compositions according to the present invention is zinc phenolsulfonate which is used at levels of from 0.1% to 4%, preferably from 0.3 % to 3% more preferably from 0.5% to 1.5% by weight.

Zinc phenolsulfonate is the substituted phenol that conforms generally to the formula: 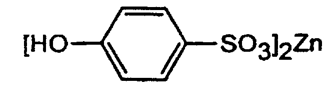 which may be commercially supplied, for example, as octahydrate crystals or powder.

Preferably, compositions of the present invention will comprise no more than 2% by weight of and especially no more than 1 % by weight of water. The levels given for the water component correspond to water in liquid form, and do not include water that may be present as part of a zinc phenolsulfonate complex.

The present liquid deodorant compositions additionally comprise a silicone emollient selected from volatile silicone emollients and/or non-volatile silicone emollients. These emollient components are valuable in the present compositions for the provision of enhanced smooth, non-sticky, dry skin feel benefits.

Generally, these emollients are present at a total level of from 0.1% to 10%, preferably from 0.5% to 5% and more preferably from 1% to 4% by weight.

The mixture of emollient materials should generally have a viscosity in the range of from about 0.65 centistokes to about 50 centistokes, as measured by a Brookfield cone and plate viscometer, at 25°C. Generally, the mixture of emollient materials must also be of a certain polarity to remain stable in the present compositions. The precise polarity will depend upon the selection and level of other components of the composition, as will be understood by those skilled in the art. Polarity of the emollients can be characterised in terms of solubility parameter. In general, the solubility parameter (units equal (ca/cm³)^{1/2}) should be less than about 10.
The solubility parameter is defined in the *Polymer Handbook* 3rd Ed. (John Wiley and Sons, New York), J Brandrup and E.H. Immergut, Chapter VII, pp. 519-559, as the square root of the cohesive energy density and describes the attractive strength between molecules of the material. The solubility parameters for the present emollient materials can be determined by surface tension measurement as outlined in Vaughan, C.D., *J. Soc. Cosmet. Chem.,* 36, 319-333, 1985. Solubility parameters may also be determined by other measurement procedures, correlation with other physical properties, or indirect measurement which provide equivalent results.

By "volatile" silicone, as is well known and understood in the art it is meant that the silicone fluids of which the volatile silicone emollient is comprised are readily vaporisable (i.e., they exhibit an appreciable vapour pressure) at ambient temperatures (particularly at about 20 to 25°C). Generally, it is considered herein that molecules with a vapour pressure of 0.1mmHg at 25°C, preferably 0.2mmHg or greater at 25°C are volatile.

Volatile silicone emollients suitable for use in the liquid deodorant compositions of the present invention have a viscosity of from about 0.65 centistokes to about 10 centistokes at 25C.

A description of volatile silicones is found in Todd and Byers, "Volatile Silicone Fluids for Cosmetics", *Cosmetics and Toiletries,* 91:27-32 (1976). Volatile silicones useful herein are also disclosed in US-A-4,874,868, Bolich, Jr., issued October 18, 1989.

Cyclic volatile siloxanes useful herein include those of the following formula: 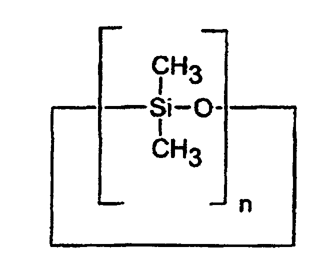 wherein n is from about 3 to about 9.

Linear volatile siloxane oils include those of the formula:
(CH₃)₃Si-O-[Si(CH₃)₂-O]ₙ-Si(CH₃)₃
wherein n is from about 1 to about 9. Linear volatile siloxanes generally have viscosities of less than about 5 centistokes at 25°C, and preferably less than about 1 centistoke at 25°C, whereas the cyclic volatile siloxanes generally have viscosities less than about 10 centistokes at 25°C. Volatile siloxanes suitable for use in the compositions to the present invention are cyclic compounds, i.e., cyclomethicones. Cyclic siloxanes are those containing about 4 or 5 silicone monomer units, i.e., D4 or D5 cyclomethicones. Examples of the volatile siloxane oils suitable for use in the present invention include Silicone 344 Fluid (RTM), Silicone 345 Fluid (RTM) (sold by Dow Corning Corporation); and Silicone SF-1173 (RTM) (sold by General Electric Company).

Non-volatile silicone emollients suitable for use herein can comprise one or more silicone fluid materials, but should have an "average" viscosity within the range of from about 1 centistoke to about 50 centistokes, preferably from about 10 centistokes to about 20 centistokes, at 25°C. By "average viscosity" is meant that the non-volatile silicone emollient can have one or more non-volatile silicone emollients outside of the specified range of about 1 to about 50 centistokes, but the overall, i.e., the weighted average viscosity should be within said range. Viscosity can be measured by a Brookfield cone and plate viscometer, or other equivalent method. By "non-volatile" silicone, as is well known and understood in the art, is meant that the silicone fluids, of which the non-volatile silicone emollient is comprised, are not readily vaporisable (i.e., they do not exhibit an appreciable vapour pressure) at ambient temperatures (particularly at about 20 to 25°C).

The non-volatile silicone emollients that may be used in the present compositions include polyalkyl siloxanes, polyalkylaryl siloxanes, and polyether siloxane copolymers, and mixtures thereof. Preferred non-volatile silicone emollients are linear polyalkyl siloxanes, especially linear polydimethyl siloxanes (i.e., dimethicone). Preferred non-volatile emollients for use have viscosities of from about 10 centistokes to about 20 centistokes at 25°C. These siloxanes are available, for example, from the General Electric Company (Silicone Products Division, Waterford, NY, USA) in the Viscasil (RTM) series and from Dow Corning Corporation (Midland, Michigan, USA), as the Dow Corning 200 Fluid series.

Other non-volatile silicone emollients that can be used include polymethylphenylsiloxanes. These siloxanes are available, for example, from the General Electric Company as SF 1075 methyl phenyl fluid (RTM) or from Dow Corning as 556 Fluid (RTM).

A polyether siloxane copolymer that may be used is, for example, a dimethyl polyoxyalkylene ether copolymer fluid. Such copolymers are available, for example, from the General Electric Company as SF-1066 organosilicone surfactant.

Preferred non-volatile silicone emollients for use are linear polydimethyl siloxanes and phenyl dimethicone. The most preferred non-volatile silicone emollient for use herein is the Dow Corning 200 Fluid series (linear polydimethyl siloxanes).

Applicant has found that the combination of polyhydric alcohol, C₁-C₄ alcohol and silicone emollient material in a weight ratio of from 1:4 : 0.05 to 4:1 : 0.05, is particularly valuable for the delivery of further emolliency benefits in combination with desirable skin feel attributes. Highly preferred herein is the combination of dipropylene glycol : ethanol : dimethicone in a weight ratio of from about 1 : 3 : 0.05 to about 1 : 1 : 0.05.

Additional, optional, auxiliary non-volatile emollients may be incorporated into the compositions of the present compositions. Auxiliary non-volatile emollients, suitable for use in the liquid deodorant compositions, are well known by those of skill in the art and include hydrocarbons, mineral oils, fatty alcohols, esters formed by the reaction of C₃-C₁₈ fatty alcohols with C₃-C₁₈ fatty acids, esters formed by the reaction of benzoic acid and C₁₂-C₁₈ alcohols, and mixtures thereof.
The preferred additional emollients include, for example, diisopropyl adipate, isopropyl myristate, isopropyl palmitate, C₁₂-C₁₅ alcohols benzoate, and mixtures thereof. The most preferred additional non-volatile emollients for use are C₁₂-C₁₅ alcohols benzoate. Other non-volatile emollients include, but are not limited to, glycerine, and polyethylene glycol (PEG) having three or more ethylene monomer units. Such materials include, for example, PEG-3 (i.e. PEG with 3 ethylene monomer units), PEG-4, PEG-6, PEG-8 and mixtures thereof. Additional, optional non-volatile emollients may be present in the compositions according to the present invention at a level of from 0.1 % to 3 %, preferably from about 0.5 % to about 2% by weight.

Perfumes, or fragrances, are generally present in the compositions hereof to mask malodors. Perfumes can also provide an aesthetically pleasing scent to the products. In the present invention, perfume is typically present at a level of at least 0.1%, preferably from 0.3 % to 6%, and more preferably from 1% to 4% by weight.

Perfumes are made by those skilled in the art in a wide variety of fragrances and strengths. Typical perfumes are described in Arctander, *Perfume and Flavour Chemicals* (Aroma Chemicals), Vol. I and II (1969); and Arctander, *Perfume and Flavour Materials of Natural Origin* (1960). The perfumes selected for use herein are chosen not only for their scent and strength, but also to meet the aesthetic demands of the consumer. Perfumes useful in the present invention are any which are suitable for use in the cosmetic industry.

Particularly preferred for use herein is masking base 55356 available from Orissa Drebing Gmbh.

The compositions of the present invention may also comprise a number of non-emollient optional components to provide cosmetic or aesthetic benefits. For example, preservatives, fillers, and thickeners may be used. Suitable thickening agents include polyethylene powder manufactured by U.S.I. Chemicals (New York, New York, USA), having a mean particle diameter of less than about 20 microns.

These optional components should be chosen so as not to unduly interfere with the efficacy and the composition stability. Such optional components are generally present in the compositions of the present invention each at a level of from 0.001% to 5% by weight, preferably at a level of from 0.001% to about 1% by weight.

The present compositions are preferably in the form of an aerosol spray. However, the present invention may be applicable to other liquid deodorant product types, such as a low-viscosity roll-on liquid or a pump-spray. Products formulated as aerosols will also comprise a propellant material. Any of the commonly used propellants in the aerosol art are suitable. The propellant can be any liquefiable gas conventionally used for aerosol containers. Examples of materials that are suitable for use as propellants are trichlorofluoromethane, dichlorofluoromethane, dimethylether e.g. Dymel 152A (RTM) supplied by Du Pont, propane, butane and isobutane, used singly or admixed and propane butane e.g. CAP 40 (RTM). Dymel 152A and propane butane are preferred.

Compressed gases such as carbon dioxide, nitrogen and air may also be used in aerosol compositions according to the present invention.

The amount of the propellant gas is governed by normal factors, as are well known in the aerosol art. The compositions described previously herein serve as the concentrate and generally comprise from 30% to 80%, preferably 40% to 75%, more preferably from 50% to 70%, of the total aerosol composition, while the propellant generally comprises from 20% to 70%, preferably from 25% to 60%, more preferably from 30% to 50%.

If a propellant such as dimethylether utilises a vapour pressure suppressant (e.g., trichloroethane or dichloromethane), the amount of suppressant is included as part of the propellant.

Although the non-volatile silicone or other silicone fluid, or fluid emollient may suitably serve as a carrier liquid in the compositions hereof, additional materials may also be used, particularly in the case of aerosol compositions. The carrier liquid can further aid efficacy by keeping the deodorant compound in contact with the skin so that it does not rub off or wash off. Examples of additional materials are alcohols such as lauryl alcohol, hexadecyl alcohol, and oleyl alcohol; carboxylic acids such as lauric and oleic acid; and lanolin and its derivatives such as acetylated lanolin.

### Method of Manufacture

The processes for making liquid deodorant compositions having long lasting odor stability, excellent cosmetics and low skin irritation, and the equipment used in such processes, are well known to those skilled in the art. Such compositions are batch processed (i.e., discrete processing steps are used).

Liquid deodorants are generally made at room temperature. Depending on the equipment and ingredients, best results may be seen when the zinc phenolsulphonate is dissolved in the ethanol and the remaining ingredients are added from most polar to least polar. Agitation is required throughout the processing to preferably achieve a homogeneous single phase end product. Aerosol propellant, if applicable, can be included according to standard industry practise.

### Method of Use

The deodorant compositions described herein are utilised in conventional ways to treat or prevent the development of malodors of the human body. Specifically, a safe and effective amount of the liquid deodorant compostion is applied topically to the body (e.g., auxillary areas) one or more times a day, preferably using an aerosol device, although any of the delivery systems for liquid deodorants may be used. When this is done, malodors are effectively prevented from developing, without sacrificing good aesthetics upon application for the user. The compositions of the present inventions provide for long lasting odor stability, excellent cosmetics, desirable non-sticky, non-greasy skin feel attributes and low skin irritiaton.

The following examples I, II and III illustrate the compositions according to the present invention. Examples IV and V illustrate compositions not according to the present invention.

| Propellant | CAP 40 |
|---|---|
| Polyhydric alcohol | Dipropylene glycol |
| C₁-C₄ alcohol | Denatured Ethanol B-100 |
| Anti-bacterial | Zinc phenolsulphonate |
| Silicone emollient | Dimethicone |

| Component | I | II | III | IV | V |
|---|---|---|---|---|---|
| Propellant | 30 | 40 | 45 | 50 | 50 |
| Polyhydric alcohol | 10 | 20 | 25 | 30 | 30 |
| Anti-bacterial | 0.5 | 1.0 | 1.5 | 2.0 | 4.0 |
| Silicone emollient | 5 | 1 | 2 | - | - |
| C₁-C₄ alcohol | to balance | | | | |

## Claims

1. A liquid deodorant composition comprising:
a) from 10% to 40% by weight, of a polyhydric alcohol selected from propylene glycol, dipropylene glycol, polypropylene glycols having three or more propylene monomer units, ethylene glycol, diethylene glycol, hexylene glycol, butylene glycol, and mixtures thereof;
b) from 10% to 70% by weight, of C₁-C₄ monohydric alcohol;
c) from 0.1% to 4% by weight, of zinc phenolsulphonate; and
d) from 0.1% to 10% by weight, of at least one emollient selected from volatile and non-volatile silicones and mixtures thereof.

2. A liquid deodorant composition according to Claim 1 wherein the weight ratio of polyhydric alcohol : C₁-C₄ monhydric alcohol is in the range of from 1 : 4 to 4 : 1.

3. A liquid deodorant composition according to any of Claims 1 or 2 wherein the polyhydric alcohol is diproplyene glycol.

4. A liquid deodorant composition according to any of Claims 1 to 3 wherein the auxilary emollient is a non-volatile silicone material selected from phenyl dimethicone, linear polydimethylsiloxanes and mixtures thereof.

5. A liquid deodorant composition according to any of Claims 1 to 4 wherein the weight ratio of polyhydric alcohol : C₁-C₄ alcohol :
auxilary emollient is in the range of from 1 : 4 :0.05 to 4 : 1 : 0.05.

6. An aerosol deodorant composition according to any of Claims 1 to 5 comprising:
a) from 10% to 40% by weight, of a polyhydric alcohol selected from propylene glycol, dipropylene glycol, polypropylene glycols having three or more propylene monomer units, ethylene glycol, diethylene glycol, hexylene glycol, butylene glycol, and mixtures thereof;
b) from 10% to 70% by weight, of C₁-C₄ monohydric alcohol;
c) from 0.1 % to 4% by weight, of zinc phenol sulphonate; and
d) from 0.1% to 10% by weight, of at least one emollient selected from volatile and non-volatile silicones and mixtures thereof.

## Patentansprüche

1. Flüssige Deodorantzusammensetzung, umfassend:
a) 10 bis 40 Gew.-% eines mehrwertigen Alkohols, ausgewählt aus Propylenglykol, Dipropylenglykol, Polypropylenglykolen, welche drei oder mehr Propylenmonomereinheiten besitzen, Ethylenglykol, Diethylenglykol, Hexylenglykol, Butylenglykol, und Mischungen davon;
b) 10 bis 70 Gew.-% eines einwertigen C₁-C₄-Alkohols;
c) 0,1 bis 4 Gew.-% Zinkphenolsulfonat; und
d) 0,1 bis 10 Gew.-% mindestens eines erweichenden Mittels, ausgewählt aus flüchtigen und nicht-flüchtigen Siliconen und Mischungen davon.

2. Flüssige Deodorantzusammensetzung nach Anspruch 1, wobei das Gewichtsverhältnis mehrwertiger Alkohol : einwertiger C₁-C₄-Alkohol im Bereich von 1:4 bis 4:1 liegt.

3. Flüssige Deodorantzusammensetzung nach irgendeinem der Ansprüche 1 oder 2, wobei der mehrwertige Alkohol ein Dipropylenglykol ist.

4. Flüssige Deodorantzusammensetzung nach irgendeinem der Ansprüche 1 bis 3, wobei das zusätzliche erweichende Mittel ein nicht-flüchtiges Siliconmaterial ist, ausgewählt aus Phenyldimethicon, linearen Polydimethylsiloxanen, und Mischungen davon.

5. Flüssige Deodorantzusammensetzung nach irgendeinem der Ansprüche 1 bis 4, wobei das Gewichtsverhältnis mehrwertiger Alkohol : C₁-C₄-Alkohol : zusätzliches erweichendes Mittel im Bereich von 1:4:0.05 bis 4:1:0.05 liegt.

6. Aerosoldeodorantzusammensetzung nach irgendeinem der Ansprüche 1 bis 5, umfassend:
a) 10 bis 40 Gew.-% eines mehrwertigen Alkohols, ausgewählt aus Propylenglykol, Dipropylenglykol, Polypropylenglykolen, welche drei oder mehr Propylenmonomereinheiten besitzen, Ethylenglykol, Diethylenglykol, Hexylenglykol, Butylenglykol, und Mischungen davon.
b) 10 bis 70 Gew.-% eines einwertigen C₁-C₄-Alkohols;
c) 0,1 bis 4 Gew.-% Zinkphenolsulfonat; und
d) 0.1 bis 10 Gew.-% mindestens eines erweichenden Mittels, ausgewählt aus flüchtigen und nicht-flüchtigen Siliconen und Mischungen davon.

## Revendications

1. Composition déodorante liquide comprenant
a) 10% à 40% en poids d'un alcool polyvalent choisi parmi le propylèneglycol, le dipropylèneglycol, les polypropylèneglycols ayant trois unités monomères de propylène ou plus, l'éthylène glycol, le diéthylèneglycol, l'hexylèneglycol, le butylèneglycol et leurs mélanges;
b) 10% à 70% en poids d'un alcool monovalent en C₁-C₄;
c) 0,1% à 4% en poids de phénolsulfonate de zinc; et
d) 0,1% à 10% en poids d'au moins un émollient choisi parmi les silicones volatiles et les silicones non volatiles et leurs mélanges.

2. Composition déodorante liquide selon la revendication 1, dans laquelle le rapport pondéral de l'alcool polyvalent à l'alcool monovalent en C₁-C₄ se situe dans la plage de 1:4 à 4:1.

3. Composition liquide déodorante selon l'une quelconque des revendications 1 ou 2, dans laquelle l'alcool polyvalent est le dipropylène glycol.

4. Composition déodorante liquide selon l'une quelconque des revendications 1 à 3, dans laquelle l'émollient auxiliaire est un matériau siliconé non volatil choisi parmi le phényldiméthicone, les polydiméthylsiloxanes linéaires et leurs mélanges.

5. Composition déodorante liquide selon l'une quelconque des revendications 1 à 4, dans laquelle le rapport pondéral de l'alcool polyvalent à l'alcool en C₁-C₄ à l'émollient auxiliaire se situe dans la plage de 1:4:0,05 à 4:1:0,05.

6. Composition déodorante en aérosol selon l'une quelconque des revendications 1 à 5, comprenant :
a) 10% à 40% en poids d'un alcool polyvalent choisi parmi le propylèneglycol, le dipropylèneglycol, les polypropylèneglycols ayant trois unités monomères de propylène ou plus, l'éthylène glycol, le diéthylèneglycol, l'hexylèneglycol, le butylèneglycol et leurs mélanges;
b) 10% à 70% en poids d'un alcool monovalent en C₁-C₄;
c) 0,1% à 4% en poids de phénolsulfonate de zinc; et
d) 0,1% à 10% en poids d'au moins un émollient choisi parmi les silicones volatiles et les silicones non volatiles et leurs mélanges.
